# EUROPÄISCHE PATENTSCHRIFT

(11) **EP 0 733 075 B1**
(45) Veröffentlichungstag und Bekanntmachung des Hinweises auf die Patenterteilung: **15.10.1997**
(21) Anmeldenummer: 95902122.1
(22) Anmeldetag: 03.12.1994
(51) Int. Cl.: C08F 26/06, A61K 7/06

(54) **VERFAHREN ZUR HERSTELLUNG VON PULVERFÖRMIGEN POLYMERISATEN AUF DER BASIS VON N-VINYLCAPROLACTAM**
PROCESS FOR PRODUCING POWDERY POLYMERS BASED ON N-VINYL CAPROLACTAMS
PROCEDE DE PRODUCTION DE POLYMERES PULVERULENTS A BASE DE N-VINYLCAPROLACTAME

(30) Priorität: 11.12.1993 DE 4342281
(43) Veröffentlichungstag der Anmeldung: 25.09.1996
(73) Patentinhaber: BASF AKTIENGESELLSCHAFT, 67056 Ludwigshafen (DE)
(72) Erfinder: BLANKENBURG, Rainer, D-67067 Ludwigshafen (DE); SANNER, Axel, D-67227 Frankenthal (DE)
(86) Internationale Anmeldenummer: EP9404027
(87) Internationale Veröffentlichungsnummer: WO9515986

(56) Entgegenhaltungen:
- EP-A- 0 130 080
- EP-A- 0 455 081
- WO-A-93/18073

## Beschreibung

Die vorliegende Erfindung betrifft ein verbessertes Verfahren zur Herstellung von pulverförmigen Polymerisaten auf der Basis von N-Vinylcaprolactam durch Polymerisation von
A) 50 bis 100 Gew.-% N-Vinylcaprolactam mit
B) 0 bis 50 Gew.-% weiterer N-Vinyllactame, Vinylheteroaromaten, Vinylester, C₁- bis C₁₀-Alkylacrylate oder -methacrylate oder einer Mischung hieraus und
C) 0 bis 5 Gew.-% Säuregruppen enthaltender monoethylenisch ungesättigter Monomere.

Von organischen Lösungsmitteln wie Alkoholen freie, feste d.h. pulverförmige Polymerisate sind insbesondere auf dem Gebiet der Haarkosmetik von starkem Interesse, da infolge der weltweit immer strenger werdenden Gesetzgebung bezüglich organischer Lösungs- und Treibmittel in Haarsprays und ähnlichen Produkten fast nur noch Polymerisate, die auf rein wäßriger Basis hergestellt wurden, verlangt werden. Außerdem weisen feste Polymerisate gegenüber Lösungen oder Dispersionen generell handhabungstechnische Vorteile auf, zu erwähnen sind hier vor allem das Problem der Kontamination von insbesondere wäßrigen Polymerisatlösungen mit Mikroorganismen während der Lagerung und das Transportproblem aufgrund des höheren Gewichtes.

Polyvinylcaprolactam und Copolymerisate hiervon mit meist kleineren Mengen von Comonomeren sind auf dem Gebiet der Haarkosmetik als Filmbildner mit hervorragenden Eigenschaften bekannt. Aber auch in anderen technischen Bereichen finden solche Polymerisate Verwendung.

Aus der Literaturstelle Journal of Applied Polymer Science, Vol. 12 (1968), S. 1835-1842 (1), ist bekannt, daß oberhalb von ca. 30 bis 35°C Polyvinylcaprolactam, welches durch Massepolymerisation oder Lösungspolymerisation in Toluol hergestellt wurde, aus seinen wäßrigen Lösungen ausfällt. Aus der US-A 4 521 404 (2) ist bekannt, daß sich Copolymerisate von N-Vinylcaprolactam, z.B. mit N-Vinylpyrrolidon, hergestellt durch Lösungspolymerisation in wasserfreiem Ethanol und Abdestillieren des Lösungsmittels, ähnlich verhalten und bei leichtem Erwärmen der entsprechenden wäßrigen Lösungen, z.B. auf ca. 55°C im Falle eines N-Vinylcaprolactam-N-Vinyl-pyrrolidon-Copolymerisates im Gewichtsverhältnis von 1:1, zumindest Trübungen ergeben. In der Regel fallen derartige Ausfällungen als gummiartige, nicht mehr handhabbare Feststoffe an. Dieses Vorurteil über eine auf diesem Wege nicht zu realisierende Bereitstellbarkeit eines pulverförmigen, gut handhabbaren N-Vinylcaprolactam-Polymerisates verhinderte bislang die Trocknung solcher Polymerisate mittels hierfür üblicher technischer Trocknungsverfahren aus wäßriger Lösung, weil hierbei diese wäßrigen Lösungen in der Regel - zumindest kurzzeitig - erwärmt werden müssen.

Aus der WO 92/17509 (3) ist bekannt, daß sich feste pulverförmige N-Vinylcaprolactam-Polymerisate, beispielsweise N-Vinylcaprolactam-Homopolymerisat oder N-Vinylcaprolactam-Acrylsäure-Copolymerisat, durch Fällungspolymerisation aus organischen Lösungsmitteln wie Alkanen und Cycloalkanen, z.B. Heptan oder Cyclohexan, herstellen lassen.

Aufgabe der vorliegenden Erfindung war es, ein Herstellungsverfahren von pulverförmigen N-Vinylcaprolactamen aus wäßriger oder wäßrig umstellbarer Lösung bereitzustellen, bei dem übliche technische Trocknungsverfahren zur Anwendung gelangen können.

Demgemäß wurde das eingangs definierte Verfahren gefunden, welches dadurch gekennzeichnet ist, daß man die Monomere A, B und C
(i) in homogener wasserhaltiger Lösung, die 10 bis 95 Gew.-%, bezogen auf das Wasser, eines wassermischbaren organischen Lösungsmittels enthält, oder in einem homogenen Gemisch aus 0 bis 90 Gew.-% Wasser und 100 bis 10 Gew.-% Methanol in Gegenwart eines radikalbildenden Initiators polymerisiert und anschließend das organische Lösungsmittel gegen Wasser austauscht oder
(ii) in wäßriger Emulsion in Gegenwart eines radikalbildenden Initiators und gegebenenfalls in Gegenwart eines Emulgators polymerisiert
und die so erhaltenen wäßrigen Polymerisatlösungen oder -dispersionen einem hierfür üblichen Trocknungsverfahren zur Erzeugung pulverförmiger Polymerisate unterwirft.

In einer bevorzugten Ausführungsform polymerisiert man bei (ii) bei Abwesenheit eines Monomeren C in Gegenwart eines Emulgators. Liegen in solch einem Fall jedoch mindestens 30 Gew.-%, bezogen auf die Gesamtmenge der Monomeren, eines weiteren N-Vinyllactams als Monomeres B vor, wird vorzugsweise auf die Mitverwendung eines Emulgators bei der Polymerisation verzichtet.

Geeignete Monomere B sind weitere N-Vinyllactame, insbesondere N-Vinylpyrrolidon und N-Vinylpiperidone, Vinylheteroaromaten, insbesondere N-Vinylimidazol und Vinylpyridine oder Vinylpyridin-N-oxide, Vinylester, insbesondere Vinylacetat und Vinylpropionat, sowie C₁- bis C₁₀-Alkyl(meth)acrylate, insbesondere Methyl-, Ethyl- oder Butylacrylat oder das entsprechende -methacrylat.

Geeignete Monomere C sind monoethylenisch ungesättigte Verbindungen, die mindestens eine Säuregruppe enthalten, z.B. eine Carboxylgruppe, eine Sulfonsäuregruppe oder eine Phosphonsäuregruppe. Verbindungen dieser Art sind beispielsweise Acrylsäure, Methacrylsäure, Dimethacrylsäure, Ethylacrylsäure, Crotonsäure, Allylessigsäure oder Vinylessigsäure. Außerdem eignen sich monoethylenisch ungesättigte Dicarbonsäure, z.B. Maleinsäure, Fumarsäure, Itaconsäure, Mesaconsäure, Methylenmalonsäure, Zitraconsäure, Maleinsäureanhydrid, Itaconsäureanhydrid und Methylenmalonsäureanhydrid, wobei die Anhydride beim Eintragen in Wasser meist zu den entsprechenden Dicarbonsäuren hydrolysieren. Als Monomere C eignen sich außerdem Sulfonsäuregruppen enthaltende Monomere, z.B. 2-Acrylamido-2-methylpropansulfonsäure, (Meth)acrylsäure-3-sulfopropylester, Vinylsulfonsäure, Methallylsulfonsäure oder Allylsulfonsäure, und Phosphonsäuregruppen aufweisende Monomere wie Vinylphosphonsäure. Die Monomeren C können entweder allein oder in Mischung miteinander eingesetzt werden.

Bevorzugte Monomere C sind Acrylsäure, Methacrylsäure, Maleinsäure, Maleinsäureanhydrid, Fumarsäure, Itaconsäure, 2-Acrylamido-2-methylpropansulfonsäure, Vinylsulfonsäure, Acryl- oder Methacrylsäure-3-sulfopropylester und Vinylphosphonsäure.

Die Monomeren C können entweder in Form der freien Säuren oder vorzugsweise in Form ihrer Alkalimetall-, Erdalkalimetall- oder Ammoniumsalze in die Polymerisation eingesetzt werden. Zweckmäßigerweise setzt man diese Salze der Monomeren C gleich als wäßrige Lösungen ein. Als Kationen sind hierbei von besonderer Bedeutung Natrium, Kalium, Magnesium und Calcium, daneben aber auch Lithium, Strontium und Barium. Die Salze der Monomeren C werden normalerweise durch übliche Neutralisation der Säuren in wäßrigem Medium hergestellt.

In einer bevorzugten Ausführungsform setzt man Polymerisate aus
A) 90 bis 100 Gew.-%, insbesondere 95 bis 100 Gew.-% N-Vinylcaprolactam mit
B) 0 bis 10 Gew.-%, insbesondere 0,1 bis 5 Gew.-% der Monomeren B und
C) 0 bis 5 Gew.-%, insbesondere 0,1 bis 3 Gew.-% der Monomeren C ein.

Insbesondere werden Copolymerisate aus
A) 95 bis 99,9 Gew.-%, insbesondere 97 bis 99,5 Gew.-% N-Vinylcaprolactam mit
C) 0,1 bis 5 Ges.-%, insbesondere 0,5 bis 3 Gew.-% der Monomeren C
bevorzugt, wobei diese gemäß Ausführungsform (i) oder gemäß Ausführungsform (ii) vorzugsweise ohne Emulgator hergestellt werden können.

Insbesondere werden weiterhin Homopolymerisate aus 100 Gew.-% N-Vinylcaprolactam A bevorzugt, wobei diese vorzugsweise gemäß Ausführungsform (i) in reinem Methanol oder vorzugsweise gemäß Ausführungsform (ii) mit Emulgator hergestellt werden können.

Erfolgt die Polymerisation gemäß Ausführungsform (i) in einer homogenen Mischung aus Wasser und einem wassermischbaren organischen Lösungsmittel, dann verwendet man vorzugsweise einen C₂- bis C4-Alkanol, insbesondere Ethanol oder Isopropanol. Dabei beträgt der Anteil an organischem Lösungsmittel, bezogen auf das Wasser, 10 bis 95 Gew.-%, vorzugsweise 25 bis 85 Gew.-%, insbesondere 50 bis 75 Gew.-%.

Die Verwendung von Wasser-Methanol-Gemischen, insbesondere von reinem Methanol, bei Ausführungsform (i) ist besonders günstig für die Herstellung von N-Vinylcaprolactam-Homopolymerisaten oder von Copolymerisaten von N-Vinylcaprolactam mit weiteren N-Vinyllactamen B, insbesondere mit N-Vinylpyrrolidon.

Bei allen beiden Ausführungsformen (i) und (ii) beträgt die Konzentration der Polymeren in der wäßrigen Reaktionsmischung üblicherweise 10 bis 70, vorzugsweise 15 bis 50 Gew.-%. Der pH-Wert der wäßrigen Lösung beträgt mindestens 6 und liegt vorzugsweise im Bereich von 7 bis 9, wobei zur Einstellung des pH-Wertes der wäßrigen Reaktionslösung in der Regel eine Alkalimetall- oder Erdalkalimetallbase eingesetzt wird. Am einfachsten ist es hierbei, die Monomeren C als wäßrige Lösungen ihrer Alkalimetall-, Erdalkalimetall- oder Ammoniumsalze mit einem pH-Wert von mindestens 6 einzusetzen. Man kann jedoch auch die Monomeren C in nicht neutralisierter Form dem Reaktionsgemisch zufügen und gleichzeitig eine mindestens äquivalente Menge einer entsprechenden Base zugeben, um den pH-Wert der Reaktionsmischung in den gewünschten Bereich von 6 oder darüber zu bringen.

Die Polymerisation erfolgt üblicherweise bei Temperaturen von 40 bis 150°C, vorzugsweise bei 60 bis 100°C, bei Normaldruck oder erhöhtem Druck, in Gegenwart von radikalbildenden Initiatoren. Diese Polymerisationsinitiatoren werden üblicherweise in Mengen von 0,01 bis 20 Gew.-%, vorzugsweise 0,05 bis 10 Gew.-%, bezogen auf die Monomeren, eingesetzt.

Als radikalbildende Initiatoren sind üblicherweise alle diejenigen Verbindungen geeignet, die bei der jeweils gewählten Polymerisationstemperatur eine Halbwertszeit von weniger als 3 Stunden aufweisen. Bevorzugt werden wasserlösliche und ionische Initiatoren, beispielsweise Wasserstoffperoxid, Natriumpersulfat, Kaliumpersulfat, Ammoniumpersulfat, 2,2'-Azobis (N,N'-dimethylenisobutyramidin)dihydrochlorid, 2,2'-Azobis(2-amidinopropan)-dihydrochlorid, 2, 2'-Azobis(dimethyl-isobutyrat), 4,4'-Azobis(4-cyanopentansäure) oder tert.-Butylperpivalat.

Weniger bevorzugt aber auch bedingt verwendbar sind nicht oder nur eingeschränkt in Wasser lösliche Polymerisationsinitiatoren wie tert.-Amylperpivalat, Dioctanoylperoxid, Dilauroylperoxid, 2,2'-Azobis(2,4-dimethylvaleronitril), Dibenzoylperoxid, tert.-Butylper-2-ethylhexanoat, tert.-Amylper-2-ethylhexanoat, tert.-Butylpermaleinat oder 2,2'-Azo-bis(isobutyronitril) sowie Redox-Initiatoren wie Schwermetallsalz-Wasserstoffperoxid-Systeme.

Die wäßrige Lösung der Polymerisationsinitiatoren wird vor der Zugabe in das Polymerisationsmedium in der Regel mit einer geeigneten Basen neutralisiert.

Das Molekulargewicht der Polymeren kann durch Zugabe von Reglern in die Reaktionsmischung kontrolliert, d.h. in den meisten Fällen erniedrigt werden. Daher wird in einer bevorzugten Ausführungsform der Erfindung bei (ii) zusätzlich ein Regler zur Kontrolle des Molekulargewichtes eingesetzt. Als Regler kommen beispielsweise niedere Alkohole wie Methanol, Ethanol, Propanol, n-Butanol, Isopropanol, Isobutanol oder Pentanol in Betracht. Als Regler für das Molekulargewicht können aber auch andere üblicherweise zu diesem Zweck eingesetzte Verbindungen, wie organische Schwefelverbindungen, beispielsweise 2-Merkaptoethanol, Butylmerkaptan, Dodecylmerkaptan, Thioessigsäure oder Thiomilchsäure, Halogenverbindungen, z.B. Tetrachlorkohlenstoff oder 1,1,1-Tribrompropan, oder Ameisensäure und ihre Derivate eingesetzt werden.

Durch geeignete Wahl von Regler, Initiator, Polymerisationstemperatur und Monomerenkonzentration wird der K-Wert des erhaltenen Polymerisats, der ein Maß für das Molekulargewicht ist, eingestellt. Die K-Werte nach Fikentscher der erhaltenen Polymerisate betragen üblicherweise 10 bis 300, vorzugsweise 15 bis 200, wobei die Messungen an einer 1 gew.-%igen wäßrigen Lösung bei 25°C vorgenommen werden (gemäß H. Fikentscher, Cellulose-Chemie, Band 13, S. 58-64 und 71-74, 1932).

Als für die Ausführungsform (ii) gegebenenfalls benötigte Emulgatoren eignen sich praktisch ohne Einschränkung alle für diesen Polymerisationstyp übliche Systeme. In Frage kommen hierbei sowohl neutrale, anionische und kationische Emulgatortypen. Solche Emulgatoren werden in der Literatur in breitem Umfang beschrieben, beispielsweise bei R. Hensch in "Ullmann's Encyclopedia of Industrial Chemistry", 5. Aufl. (1987), Band A9, S. 297-339.

Beispiele für gut einsetzbare Emulgatoren sind anionenaktive Verbindungen, insbesondere Alkalimetall-, Ammonium- und Aminsalze von längerkettigen Fettsäuren, Alkalimetallsalze der Schwefelsäureester von Fettalkoholen und Fettalkoholethern, z.B. Natriumlaurylethersulfat mit 1 bis 15 mol Ethylenoxid, und von Alkylphenolen, Alkalimetallsalze von Sulfobernsteinsäuredialkylestern sowie Alkalimetallsalze der Sulfonsäuren von Alkylbenzolen, z.B. Natriumdodecylsulfat, Alkylnaphthalinen und von Naphthalin. Es können aber auch kationenaktive Verbindungen verwendet werden, beispielsweise Fettamine, quartäre Ammoniumverbindungen oder quaternisierte Pyridine, Morpholine oder Imidazoline. Gut eignen sich weiterhin auch mit 2 bis 30 ml Ethylenoxid umgesetzte Polyolfettsäureester, z.B. Sorbitan-Monooleat mit 20 mol Ethylenoxid. Oft setzt man Mischungen von verschiedenartigen Emulgatoren ein.

Bei Anwesenheit von Monomeren C ist eine Emulgatorzugabe im Prinzip nicht erforderlich, da die ionischen Gruppen in den Monomeren C schon Emulgatoreigenschaften bei den Monomeren bzw. den hieraus gebildeten Polymerisaten bewirken. Eine zusätzliche Zugabe eines üblichen Emulgators kann in solch einem Fall jedoch nicht schaden und bewirkt gegebenenfalls sogar einen besseren Ablauf des Polymerisationsvorganges.

Die Durchführung der Polymerisation erfolgt üblicherweise in Rührkesseln mit Anker-, Blatt-, Impeller- oder Mehrstufenimpulsgegenstromrührern. Soll bei hohen Konzentrationen gearbeitet werden, ist es zweckmäßig, Kneter oder ähnliche Apparate zu verwenden. Bei der Polymerisation kann der gesamte Ansatz im Reaktor vorgelegt und nach Erhitzen auf die gewünschte Temperatur durch Zugabe des Initiators die Reaktion gestartet werden. Bei dieser Fahrweise ist jedoch die Abführung der Polymerisationswärme problematisch. Zweckmäßigerweise legt man daher das Lösungsmittel im Reaktor vor und dosiert bei der gewählten Polymerisationstemperatur die Monomeren unverdünnt oder als wäßrige Monomer-Lösung sowie den Initiator als Lösung in Wasser oder Alkohol absatzweise oder kontinuierlich zu. Oft ist es auch für den Reaktionsablauf vorteilhaft, das Lösungsmittel im Reaktor vorzulegen und die Monomeren, den Initiator und das Neutralisationsmittel zur Aufrechterhaltung des erforderlichen pH-Wertes unter den Polymerisationsbedingungen zuzudosieren.

Der Austausch von organischem Lösungsmittel gegen Wasser bei Ausführungsform (i) erfolgt normalerweise durch Zugabe der entsprechenden Wassermenge zum Reaktionsansatz nach erfolgter Polymerisation und gleichzeitigem oder nachfolgendem Abdestillieren des Lösungsmittels bei Normaldruck oder bei vermindertem Druck. Besonders günstig ist die Abtrennung des organischen Lösungsmittels durch Wasserdampfdestillation.

Als Trocknungsverfahren zur Erzeugung pulverförmiger Polymerisate kommen alle solche in Frage, die zur Trocknung aus wäßriger Lösung geeignet sind. Bevorzugte Verfahren sind die Sprühtrocknung, die Sprühwirbelschichttrocknung, die Walzentrocknung und die Bandtrocknung, weniger bevorzugte aber auch anwendbare Verfahren sind die Gefriertrocknung und die Gefrierkonzentrierung.

Die durch Trocknung aus wäßriger Lösung erfindungsgemäß erhältlichen Polymerisatpulver sind in Alkoholen, Wasser und deren Mischungen löslich und weisen die bekannten vorzüglichen Eigenschaften als Hilfsmittel in haarkosmetischen Zubereitungen, z.B. als Haarlack- oder Haarsprayadditiv, auf.

Weiterhin können die erfindungsgemäß hergestellten Polymerisatpulver als Bindemittel für den Transferdruck, als Schmiermitteladditive, als Strukturverbesserer für Ackerböden sowie als Hilfsstoffe für die Agrochemie beim Saatgutcoating, bei slow-release-Düngemittelformulierungen und bei Formulierungen für die Landwirtschaft mit Adhäsionseigenschaften, als Rostverhinderer oder Rostentferner von metallischen Oberflächen, als Kesselsteinverhinderer oder Kesselsteinentferner, als Hilfsmittel bei der Erdölgewinnung aus ölhaltigem Wasser und bei der Erdöl- und Erdgasförderung und dem Erdöl- und Erdgastransport, als Wirkstofffreisetzer in pharmazeutischen Zubereitungen, als Reinigungsmittel von Abwässern, als Klebrohstoffe, als Waschmitteladditive sowie als Hilfsmittel in der Photoindustrie, in Immunochemikalien und in hautkosmetischen Zubereitungen verwendet werden.

Die im Rahmen der vorliegenden Erfindung beschriebenen Homo- und Copolymerisate des Vinylcaprolactams weisen das Phänomen der Ausfällung bei erhöhter Temperatur nicht mehr auf, sie bilden vielmehr bei Temperaturen > 35°C einen stabilen Latex, der beim Abkühlen unter diese Temperatur voll reversibel wieder in eine klare Lösung übergeht. Damit sind diese Polymerisate der Trocknung mittels konventioneller Trocknungsverfahren sehr leicht zugänglich.

### Beispiele

Die folgenden Beispiele dienen der Erläuterung der Erfindung. Der K-Wert wurde jeweils an einer 1 gew.-%igen Lösung in Wasser bei 25°C nach Fikentscher gemessen.

### Beispiel 1

Eine Lösung von 5 g 2-Acrylamido-2-methylpropansulfonsäure in 50 g Wasser wurde mit verdünnter Natronlauge auf pH 7 eingestellt. Durch Zugabe von 150 g Ethanol und 395 g N-Vinylcaprolactam wurde eine homogene Lösung erhalten (Zulauf 1). Aus 2 g tert.-Butylperpivalat und 85 g Ethanol wurde Zulauf 2 hergestellt.

In einem 2-l-Rührbehälter, der mit Rührer, Heizung, Rückflußkühler, Dosiervorrichtung, Gasein- und -auslaß ausgestattet war, wurden eine homogene Mischung aus 100 g N-Vinylcaprolactam, 100 g Ethanol, 10 g Wasser und 0,5 g tert.-Butylperpivalat vorgelegt und unter Rühren und Einleiten von Stickstoff auf 75°C erhitzt. Nach Anspringen der Polymerisation, erkennbar an einer Viskositätserhöhung der Vorlage, wurden die Zuläufe 1 und 2 gleichzeitig in 3 Stunden zugefahren. Zur Restmonomereliminierung wurde eine Nachpolymerisation durchgeführt, dazu wurde eine Lösung von 4 g tert.-Butylperpivalat in 100 g Ethanol in einem Zeitraum von 8 h bei 75°C zugefahren und anschließend noch 3 Stunden bei dieser Temperatur gehalten. Zur Herstellung der rein wäßrigen Lösung wurden 500 g Wasser zugefahren und durch Einleiten von Wasserdampf der Ethanolgehalt bei zuletzt 100°C Innentemperatur abdestilliert. Man erhielt einen weißen, niederviskosen Polymerlatex, der beim Abkühlen in eine klare, viskose Polymerlösung überging.

Die Lösung hatte einen Feststoffgehalt von 35 Gew.-% und einen Rest-N-Vinylcaprolactam-Gehalt von < 50 ppm. Der K-Wert des Polymeren betrug 34. Durch Sprühtrocknung wurde ein weißes, geruchloses Pulver erhalten.

### Beispiele 2 und 3

In Analogie zu Beispiel 1 wurden als neutralisierte Comonomere jeweils 2 Gew.-% Acrylsäure oder Methacrylsäure zusammen mit 98 Gew.-% N-Vinylcaprolactam eingesetzt. Nach der Sprühtrocknung wurden weiße Pulver mit einem K-Wert im Bereich von 30 bis 40 erhalten.

### Beispiel 4

Eine Lösung von 4 g 2-Acrylamido-2-methylpropansulfonsäure, 0,5 g 4,4'-Azobis(4-cyanopentansäure) und 2 g Thioglykolsäure in 100 g Wasser wurde mit verdünnter Natronlauge auf pH 7 eingestellt (Zulauf 1).

Zur Polymerisation wurde ein 2-1-Rührbehälter eingesetzt, der mit Rührer, Heizung, Rückflußkühler, Dosiervorrichtung, Gasein- und -auslaß ausgestattet war. Als Vorlage diente eine Lösung von 2 g 2-Acrylamido-2-methylpropansulfonsäure, 0,5 g 4,4'-Azobis-(4-cyanopentansäure) und 1 g Thioglykolsäure in 600 g Wasser, die mit verdünnter Natronlauge auf pH 7 eingestellt wurde. Durch Zugabe von 100 g geschmolzenem N-Vinylcaprolactam wurde unter intensivem Rühren eine Emulsion erhalten. Diese wurde unter Einleiten von Stickstoff auf 90°C erhitzt. Nach Anspringen der Polymerisation, erkennbar an der exothermen Reaktion, wurden gleichzeitig Zulauf 1 und 200 g geschmolzenes N-Vinylcaprolactam in 3 Stunden zugefahren.

Zur Restmonomereliminierung wurde eine Nachpolymerisation durchgeführt, dazu wurde eine Lösung des Natriumsalzes von 0,5 g 4,4'-Azobis(4-cyanopentansäure) in 100 g Wasser in einem Zeitraum von 8 Stunden bei 90°C zugefahren und anschließend noch 3 Stunden bei dieser Temperatur gehalten. Zur weiteren Restmonomerreduzierung wurde eine Wasserdampfdestillation durchgeführt. Man erhielt einen weißen, niederviskosen Polymerlatex, der beim Abkühlen in eine klare, viskose Polymerlösung überging.

Die Lösung hatte einen Feststoffgehalt von 25 % und einen Rest-N-Vinylcaprolactam-Gehalt von < 50 ppm. Der K-Wert des Polymeren betrug 33. Durch Sprühtrocknung wurde ein weißes, geruchloses Pulver erhalten.

### Beispiele 5 und 6

In Analogie zu Beispiel 1 wurden als neutralisierte Comonomere jeweils 2 Gew.-% Acrylsäure oder Methacrylsäure zusammen mit 98 Gew.-% N-Vinylcaprolactam eingesetzt. Nach der Sprühtrocknung wurden weiße Pulver mit einem K-Wert im Bereich 25 bis 40 erhalten. Durch Variation der verwendeten Menge an Regler ließen sich Polymerisate im K-Wert-Bereich 15 bis 200 erhalten.

### Beispiel 7

Eine Lösung von 1 g 4,4'-Azobis(4-cyanopentansäure) in 50 g Wasser wurde mit verdünnter Natronlauge auf pH 7 eingestellt (Zulauf 1).

Zur Polymerisation wurde ein 2-1-Rührbehälter eingesetzt, der mit Rührer, Heizung, Rückflußkühler, Dosiervorrichtung, Gasein- und -auslaß ausgestattet war. Als Vorlage diente eine Lösung von 0,5 g 4,4'-Azobis(4-cyanopentansäure) und 3,5 g Natriumdodecylsulfat in 600 g Wasser, die mit verdünnter Natronlauge auf pH 7 eingestellt wurde. Durch Zugabe von 100 g geschmolzenem N-Vinylcaprolactam wurde unter intensivem Rühren eine Emulsion erhalten. Diese wurde unter Einleiten von Stickstoff auf 90°C erhitzt. Nach Anspringen der Polymerisation, erkennbar an der exothermen Reaktion, wurden gleichzeitig Zulauf 1 und eine Mischung aus 200 g geschmolzenem N-Vinylcaprolactam und 6,5 g Tween® 80 (mit 20 mol Ethylenoxid umgesetztes Sorbitan-Monooleat der Firma Atlas-Chemie) in 3 Stunden zugefahren.

Zur Restmonomereliminierung wurde eine Nachpolymerisation durchgeführt, dazu wurde eine Lösung des Natriumsalzes von 0,5 g 4,4'-Azobis(4-cyanopentansäure) in 100 g Wasser in einem Zeitraum von 8 Stunden bei 90°C zugefahren und anschließend noch 3 Stunden bei dieser Temperatur gehalten. Zur weiteren Restmonomerreduzierung wurde eine Wasserdampfdestillation durchgeführt. Man erhielt einen weißen, niederviskosen Polymerlatex, der beim Abkühlen in eine klare, viskose Polymerlösung überging.

Die Lösung hatte einen Feststoffgehalt von 35 % und einen Rest-N-Vinylcaprolactam-Gehalt von 100 ppm. Der K-Wert des Polymeren betrug 45. Durch Sprühtrocknung wurde ein weißes, geruchloses Pulver erhalten.

### Beispiel 8

Eine Lösung von 5 g 4,4'-Azobis(4-cyanopentansäure) in 500 g Wasser wurde mit verdünnter Natronlauge auf pH 7 eingestellt (Zulauf 1).

Zur Polymerisation wurde ein 3-1-Rührbehälter eingesetzt, der mit Rührer, Heizung, Rückflußkühler, Dosiervorrichtung, Gasein- und -auslaß ausgestattet war. Die Polymerisation wurde im batch durchgeführt, dazu wurde eine Mischung aus 1500 g Wasser, 70 g Zulauf 1, 400 g N-Vinylpyrrolidon und 400 g geschmolzenem N-Vinylcaprolactam hergestellt. Durch intensives Rühren wurde eine Suspension erhalten und diese unter Einleiten von Stickstoff auf 90°C Innentemperatur erhitzt. Nach Anspringen der Polymerisation, erkennbar an der exothermen Reaktion und dem Viskositätsanstieg des Polymerisationsmediums, wurde Zulauf 1 in 5 Stunden zugefahren. Während der Polymerisation wurde mit ca. 1500 g Wasser verdünnt. Zur Restmonomerreduzierung wurde anschließend noch 3 Stunden bei 90°C gehalten. Zur weiteren Restmonomerreduzierung wurde eine Wasserdampfdestillation durchgeführt. Man erhielt einen weißen Polymerlatex, der beim Abkühlen in eine klare, hochviskose Polymerlösung überging.

Die Lösung hatte einen Feststoffgehalt von ca. 19 Gew.-% und einen Rest-N-Vinylcaprolactam-Gehalt von 400 ppm. Der K-Wert des Polymeren betrug 83. Durch Sprühtrocknung aus stark verdünnter Lösung oder durch Walzentrocknung wurde ein weißes, geruchloses Pulver erhalten.

### Beispiel 9

In Analogie zu Beispiel 8 wurde ein Gemisch aus N-Vinylpyrrolidon und N-Vinylcaprolactam im Gewichtsverhältnis 1:1 in homogener Lösung in Methanol mit dem Polymerisationsstarter t-Butylperpivalat polymerisiert. Nach Austausch des Lösungsmittels gegen Wasser wurde ein weißer Latex erhalten, der beim Abkühlen in eine viskose, klare Lösung überging.

Die Lösung hatte einen Feststoffgehalt von ca. 37 Gew.-% und einen Rest N-Vinylcaprolactam-Gehalt von 500 ppm. Der K-Wert des Polymeren lag im Bereich von 60 bis 70. Durch Sprühtrocknung aus stark verdünnter Lösung oder durch Walzentrocknung wurde ein weißes, geruchloses Pulver erhalten.

## Patentansprüche

1. Verfahren zur Herstellung von pulverförmigen Polymerisaten auf der Basis von N-Vinylcaprolactam durch Polymerisation von
A) 50 bis 100 Gew.-% N-Vinylcaprolactam mit
B) 0 bis 50 Gew.-% weiterer N-Vinyllactame, Vinylheteroaromaten, Vinylester, C₁- bis C₁₀-Alkylacrylate oder -methacrylate oder einer Mischung hieraus und
C) 0 bis 5 Gew.-% Säuregruppen enthaltender monoethylenisch ungesättigter Monomere,
dadurch gekennzeichnet, daß man die Monomere A, B und C
(i) in homogener wasserhaltiger Lösung, die 10 bis 95 Gew.-%, bezogen auf das Wasser, eines wassermischbaren organischen Lösungsmittels enthält, oder in einem homogenen Gemisch aus 0 bis 90 Gew.-% Wasser und 100 bis 10 Gew.-% Methanol in Gegenwart eines radikalbildenden Initiators polymerisiert und anschließend das organische Lösungsmittel gegen Wasser austauscht oder
(ii) in wäßriger Emulsion in Gegenwart eines radikalbildenden Initiators und gegebenenfalls in Gegenwart eines Emulgators polymerisiert
und die so erhaltenen wäßrigen Polymerisatlösungen oder -dispersionen einem hierfür üblichen Trocknungsverfahren zur Erzeugung pulverförmiger Polymerisate unterwirft.

2. Verfahren nach Anspruch 1, dadurch gekennzeichnet, daß man bei Ausführungsform (ii) bei Abwesenheit eines Monomeren C in Gegenwart eines Emulgators polymerisiert.

3. Verfahren nach Anspruch 2, dadurch gekennzeichnet, daß man bei Ausführungsform (ii) bei Abwesenheit eines Monomeren C und in Gegenwart von mindestens 30 Gew.-%, bezogen auf die Gesamtmenge der Monomeren, eines weiteren N-Vinyllactams als Monomerem B in Abwesenheit eines Emulgators polymerisiert.

4. Verfahren nach den Ansprüchen 1 bis 3 unter Verwendung der Monomeren C in Form ihrer Alkalimetall-, Erdalkalimetall- oder Ammoniumsalze.

5. Verfahren nach den Ansprüchen 1 bis 4, dadurch gekennzeichnet, daß man bei der Polymerisation gemäß Ausführungsform (ii) zusätzlich einen Regler zur Kontrolle des Molekulargewichtes einsetzt.

6. Verfahren nach den Ansprüchen 1 bis 5 unter Verwendung von N-Vinylpyrrolidon, N-Vinylpiperidonen, N-Vinylimidazol, Vinylpyridinen oder Vinylpyridin-N-Oxiden, Vinylacetat, Vinylpropionat, Methyl-, Ethyl- oder Butylacrylat oder dementsprechenden -methacrylat als Monomere B.

7. Verfahren nach den Ansprüchen 1 bis 6 unter Verwendung von Acrylsäure, Methacrylsäure, Maleinsäure, Maleinsäureanhydrid, Fumarsäure, Itaconsäure, 2-Acrylamido-2-methylpropansulfonsäure, Vinylsulfonsäure, Acryl- oder Methacrylsäure-3-sulfopropylester oder Vinylphosphonsäure als Monomere C.

8. Verfahren nach den Ansprüchen 1 bis 7 durch Polymerisation von
A) 90 bis 100 Gew.-% N-Vinylcaprolactam mit
B) 0 bis 10 Gew.-% der Monomeren B und
C) 0 bis 5 Gew.-% der Monomeren C.

9. Verfahren nach den Ansprüchen 1, 4, 5 oder 7 durch Copolymerisation von
A) 95 bis 99,9 Gew.-% N-Vinylcaprolactam mit
C) 0,1 bis 5 Gew.-% der Monomeren C.

10. Verfahren nach den Ansprüchen 1, 2 oder 5 durch Homopolymerisation von N-Vinylcaprolactam A.

11. Verfahren nach den Ansprüchen 1 bis 10, dadurch gekennzeichnet, daß man als Trocknungsverfahren zur Erzeugung pulverförmiger Polymerisate die Sprühtrocknung, die Sprühwirbelschichttrocknung, die Walzentrocknung, die Bandtrocknung, die Gefriertrocknung oder die Gefrierkonzentrierung verwendet.

12. Verwendung von gemäß Anspruch 1 bis 11 hergestellten pulverförmigen Polymerisaten als Hilfsmittel in haarkosmetischen und in hautkosmetischen Zubereitungen.

## Claims

1. A process for preparing a pulverulent polymer based on N-vinylcaprolactam by polymerizing
A) from 50 to 100% by weight of N-vinylcaprolactam with
B) from 0 to 50% by weight of further N-vinyllactams, heteroaromatic vinyl compounds, vinyl esters, C₁-C₁₀-alkyl acrylates or methacrylates, or a mixture thereof, and
C) from 0 to 5% by weight of monoethylenically unsaturated monomers containing acid groups,
which comprises subjecting the monomers A, B and C
(i) to polymerization in homogeneous, water-containing solution, which contains from 10 to 95% by weight of a water-miscible organic solvent, based on the water, or in a homogeneous mixture comprising from 0 to 90% by weight of water and from 100 to 10% by weight of methanol, in the presence of a free-radical initiator, and then replacing the organic solvent by water, or
(ii) to polymerization in aqueous emulsion in the presence of a free-radical initiator and in the presence or absence of an emulsifier
and subjecting the resulting aqueous polymer solutions or dispersions to a drying technique which is customary for such formulations, in order to produce a pulverulent polymer.

2. A process as claimed in claim 1, wherein polymerization in embodiment (ii) is carried out in the absence of a monomer C but in the presence of an emulsifier.

3. A process as claimed in claim 2, wherein polymerization in embodiment (ii) is carried out in the absence of a monomer C and in the presence of at least 30% by weight, based on the overall quantity of the monomers, of a further N-vinyllactam as monomer B in the absence of an emulsifier.

4. A process as claimed in any of claims 1 to 3 using the monomers C in the form of their alkali metal, alkaline earth metal or ammonium salts.

5. A process as claimed in any of claims 1 to 4, wherein the polymerization in accordance with embodiment (ii) is carried out with the additional use of a regulator to control the molecular weight.

6. A process as claimed in any of claims 1 to 5 using N-vinylpyrrolidone, N-vinylpiperidones, N-vinylimidazole, vinylpyridines or vinylpyridine N-oxides, vinyl acetate, vinyl propionate, methyl, ethyl or butyl acrylate or a corresponding methacrylate as monomers B.

7. A process as claimed in any of claims 1 to 6 using acrylic acid, methacrylic acid, maleic acid, maleic anhydride, fumaric acid, itaconic acid, 2-acrylamido-2-methylpropanesulfonic acid, vinylsulfonic acid, 3-sulfopropyl acrylate or methacrylate, or vinylphosphonic acid as monomers C.

8. A process as claimed in any of claims 1 to 7, which comprises polymerizing
A) from 90 to 100% by weight of N-vinylcaprolactam with
B) from 0 to 10% by weight of the monomers B and
C) from 0 to 5% by weight of the monomers C.

9. A process as claimed in any of claims 1, 4, 5 and 7, which comprises copolymerizing
A) from 95 to 99.9% by weight of N-vinylcaprolactam with
C) from 0.1 to 5% by weight of the monomers C.

10. A process as claimed in any of claims 1, 2 and 5, which comprises homopolymerizing N-vinylcaprolactam A.

11. A process as claimed in any of claims 1 to 10, wherein the drying technique used to produce a pulverulent polymer is spray drying, fluidized-bed spray drying, roller drying, belt drying, freeze drying or freeze concentrating.

12. The use of a pulverulent polymer prepared as claimed in any of claims 1 to 11 as an auxiliary in cosmetic hair preparations or cosmetic skin preparations.

## Revendications

1. Procédé de préparation de polymères pulvérulents, à base de N-vinylcaprolactame, par la polymérisation de
A) 50 à 100% en poids de N-vinylcaprolactame avec
B) 0 à 50% en poids d'autres N-vinyllactames, de composés vinylhétéroaromatiques, d'esters vinyliques, d'acrylates d'alkyle en C₁ à C₁₀ ou de méthacrylates d'alkyle en C₁ à C₁₀, ou d'un mélange de ces composés et
C) 0 à 5% en poids de monomères monoéthyléniquement insaturés contenant des radicaux acide,
caractérisé en ce que l'on polymérise les monomères A, B et C
(i) dans une solution aqueuse homogène, qui contient de 10 à 95% en poids, par rapport à l'eau, d'un solvant organique miscible à l'eau, ou dans un mélange homogène constitué de 0 à 90% en poids d'eau et de 100 à 10% en poids de méthanol, en présence d'un amorceur générateur de radicaux et on échange ensuite le solvant organique contre de l'eau, ou bien
(ii) dans une émulsion aqueuse, en présence d'un amorceur générateur de radicaux et éventuellement en présence d'un émulsif
et on soumet les dispersions ou les solutions aqueuses de polymère ainsi obtenues à un procédé de séchage usuel, à cette fin, en vue de l'obtention de polymères pulvérulents.

2. Procédé suivant la revendication 1, caractérisé en ce que l'on opère la polymérisation dans le cas de la forme de réalisation (ii) et de l'absence d'un monomère C, en présence d'un émulsif.

3. Procédé suivant la revendication 2, caractérisé en ce que l'on opère la polymérisation dans le cas de la forme de réalisation (ii) et de l'absence d'un monomère C et en présence d'au moins 30% en poids, par rapport à la quantité totale des monomères, d'un N-vinyllactame supplémentaire, à titre de monomère B, en l'absence d'un émulsif.

4. Procédé suivant l'une quelconque des revendications 1 à 3, caractérisé en ce que l'on utilise les monomères C, sous forme de leurs sels de métaux alcalins, de métaux alcalino-terreux ou d'ammonium.

5. Procédé suivant l'une quelconque des revendications 1 à 4, caractérisé en ce que dans le cas de la polymérisation selon la forme de réalisation (ii), on utilise complémentairement un régulateur pour la maîtrise du poids moléculaire.

6. Procédé suivant l'une quelconque des revendications 1 à 5, en recourant à l'emploi de N-vinylpyrrolidone, de N-vinylpipéridones, de N-vinylimidazole, de vinylpyridines, ou de N-oxydes de vinylpyridines, d'acétate de vinyle, de propionate de vinyle, d'acrylate de méthyle, d'éthyle ou de butyle, ou des méthacrylates correspondants, à titre de monomères B.

7. Procédé suivant l'une quelconque des revendications 1 à 6, en recourant à l'emploi d'acide acrylique, d'acide méthacrylique, d'acide maléique, d'anhydride de l'acide maléique, d'acide fumarique, d'acide itaconique, d'acide 2-acrylamido-2-méthylpropanesulfonique, d'acide vinylsulfonique, d'esters 3-sulfopropyliques de l'acide acrylique ou de l'acide méthacrylique, ou d'acide vinylphosphonique, à titre de monomères C.

8. Procédé suivant l'une quelconque des revendications 1 à 7, par la polymérisation de
A) 90 à 100% en poids de N-vinylcaprolactame avec
B) 0 à 10% en poids des monomères B et
C) 0 à 5% en poids des monomères C.

9. Procédé suivant les revendications 1, 4, 5 ou 7, par la copolymérisation de
A) 95 à 99,9% en poids de N-vinylcaprolactame avec
C) 0,1 à 5% en poids des monomères C.

10. Procédé suivant les revendications, 1, 2 ou 5, par l'homopolymérisation du N-vinylcaprolactame A.

11. Procédé suivant l'une quelconque des revendications 1 à 10, caractérisé en ce que l'on recourt, à titre de procédé de séchage, pour l'obtention de polymères pulvérulents, à un séchage par pulvérisation, à un séchage par pulvérisation en couche fluidisée, à un séchage en tambour, à un séchage sur bande, à un séchage par congélation ou à une concentration par congélation.

12. Utilisation des polymères pulvérulents préparés suivant l'une quelconque des revendications 1 à 11, à titre d'adjuvants, dans des préparations cosmétiques, pour les soins des cheveux et de la peau.
